# EUROPÄISCHE PATENTANMELDUNG

(11) **EP 1 777 290 A1**
(43) Veröffentlichungstag der Anmeldung: **25.04.2007**
(21) Anmeldenummer: 05022746.1
(22) Anmeldetag: 19.10.2005
(51) Int. Cl.: C12M 1/107, C12M 1/113, C12P 5/02

(54) **Biogasanlagen-Betriebsverfahren zum einstufigen Betrieb**

(71) Anmelder: U.T.S. Umwelt-Technik-Süd GmbH, 84419 Obertaufkirchen (DE)
(72) Erfinder: Bürger, Adam, 84405 Grüntegernbach (DE)
(74) Vertreter: Neubauer, Hans-Jürgen

(57) **Zusammenfassung**

Die Erfindung betrifft ein Biogasanlagen-Betriebsverfahren zum einstufigen Betrieb einer Biogasanlage zur Erzeugung von Biogas durch anaerobe Nassvergärung aus einem dem Biogasfermenter der Biogasanlage zugeführten Substrat einer vorgegebenen Substratzusammensetzung. Erfindungsgemäß erfolgt in einer Anfahrphase mit dem Substrat (S1) der vorgegebenen Substratzusammensetzung, als Neustart nach einer Neubeschickung oder im eingefahrenen Betrieb nach einer Änderung der Substratzusammensetzung, eine Zudosierung von substratspezifischen Bakterien, die unabhängig von der Biogasanlage in einem Züchtungsreaktor (4; 6a) mittels eines Substrats (S1) der vorgegebenen Substratzusammensetzung vorgezüchtet worden sind. Dadurch wird die Anfahrphase in Verbindung mit einer höheren Biogasausbeute verkürzt.

## Beschreibung

Die Erfindung betrifft ein Biogasanlagen-Betriebsverfahren zum einstufigen Betrieb einer Biogasanlage nach dem Oberbegriff des Anspruchs 1.

Die Biogasgewinnung und -nutzung zur Energieerzeugung gewinnt, vorrangig im landwirtschaftlichen Bereich, zunehmend an Bedeutung, insbesondere durch die staatlich festgelegten Vergütungssätze für elektrischen Strom aus regenerativen Energien.

Eine Biogasanlage für einen kontinuierlichen, einstufigen Betrieb besteht im wesentlichen aus einem Biogasfermenter, in dem in einem anaeroben Nassvergärungsprozess zur Gewinnung von Biogas organisches Substrat vergoren wird. Je nach den Gegebenheiten können dem Biogasfermenter ein Nachfermenter und ein Endlager sowie ein Gasspeicher für das erzeugte Biogas nachgeordnet sein. Für flüssige Substratbestandteile sind Pumpen vorgesehen, mit denen auch ein Umpumpen zwischen den einzelnen Behältern möglich ist. Für feste Biomassen sind Feststoffzudosiereinrichtungen mit Wägeeinrichtungen und Befüllschnecken bekannt. Weiter sind in den Behältern und Fermentern Rühreinrichtungen angebracht.

Die Erzeugung von Biogas erfolgt bei der anaeroben Nassvergärung im wesentlichen in vier Schritten:
Im ersten Schritt, der Hydrolyse, werden komplexe Verbindungen im organischen Substrat in einfachere organische Verbindungen zerlegt. Daran beteiligte Bakterien setzen hierzu Enzyme frei, die das Material auf biochemischem Wege zersetzen.

Die gebildeten Zwischenprodukte werden im zweiten Schritt, der Versäuerungsphase (Acidogenese), durch säurebildende Bakterien weiter zu niederen Fettsäuren sowie Kohlendioxid und Wasserstoff abgebaut.

Im dritten Schritt, der Essigsäurebildung (Acetogenese), werden diese Produkte wiederum durch Bakterien zu Vorläufersubstanzen des Biogases umgesetzt. Für diese Bakterien ist ein hoher Wasserstoffgehalt schädlich, so dass sie mit Methanbakterien aus dem nachfolgenden vierten Schritt eine enge Lebensgemeinschaft bilden müssen, da diese bei der Bildung von Methan Wasserstoff verbrauchen.

Im vierten und letzten Schritt, der Methanogenese, wird aus den Produkten der Acetogenese von den Methanbakterien Methan gebildet. Dadurch ergibt sich im Biogasfermenter oberhalb des Substratspiegels Biogas mit einer Zusammensetzung von etwa 50 bis 75 Vol.-% Methan, 25 bis 45 Vol.-% Kohlendioxid, 2 bis 7 Vol.-% Wasser sowie weiterer Anteile in geringer Konzentration.

Im vorliegenden einstufigen Verfahren laufen die vorstehenden vier Abbauschritte gemeinsam in einem Biogasfermenter ab. Für die einzelnen Abbaustufen sind unterschiedliche Bakterien erforderlich, mit unterschiedlichen Anforderungen an die Milieubedingungen ihres Lebensraums, so dass bei einer einstufigen Anlage ein Kompromiss bezüglich des Lebensraums für alle beteiligten Bakterien gefunden werden muss. Die Methanbakterien sind dabei am empfindlichsten gegenüber Störungen und vermehren sich vergleichsweise nur langsam, so dass die Milieubedingungen im Fermenter im wesentlichen den Methanbakterien anzupassen sind.

Ein wesentlicher Aspekt der Biogasgewinnung ist die Nutzung zur Einspeisung in ein Blockheizkraftwerk für eine wirtschaftlich lukrative Energieerzeugung. Dort wird in einem Gasmotor Biogas verbrannt, dessen Energiegehalt in mechanische Drehenergie und anschließend durch einen Generator in elektrische Energie zur weiteren Verwertung umgewandelt wird.

Für eine angestrebte hohe Biogaserzeugungsrate und davon abhängige hohe Erzeugungsrate von elektrischer Energie ist es erforderlich die Milieubedingungen für die Bakterien möglichst optimal zu gestalten. Dazu wird die Temperatur im Biogasfermenter je nach Verfahrensführung auf einen mesophilen Temperaturbereich, etwa zwischen 32 und 42°C oder einen thermophilen Temperaturbereich, etwa zwischen 50 und 57°C geregelt, wodurch sich vorrangig mesophile oder thermophile Bakterienkulturen bilden und vermehren. Die Nährstoffversorgung für die Bakterien wird durch eine bestimmte vorgegebene meist chargenförmige Beschickung mit Substrat vorgenommen. Weiter ist es erforderlich den pH-Wert zu überwachen und gegebenenfalls zu korrigieren. Zudem ist es erforderlich, den Fermenterinhalt geeignet zu durchmischen, um einen intensiven Kontakt von Bakterien und Substrat zu ermöglichen. Weiter ist die Biogaserzeugungsrate auch abhängig von der hydraulischen Verweilzeit des Substrats im Fermenter. Im eingefahrenen kontinuierlichen Betrieb einer Biogasanlage mit jeweils etwa gleicher Substratbeschickung wird bisher eine Prozessoptimierung durch Anpassungen und Optimierungen der vorstehenden Parameter durchgeführt. Wie viel Biogas aber letztlich in einer Biogasanlage produziert wird, hängt im wesentlichen von der Zusammensetzung und dem Energieinhalt der eingesetzten Substrate ab.

Der Betreiber einer Biogasanlage kann aber regelmäßig die Zusammensetzung der Substrate nicht frei bestimmen, sondern ist abhängig von der gegebenenfalls saisonalen Verfügbarkeit nachwachsender Biomasse und/oder von Wirtschaftsdüngern und/oder von zugelieferter Biomasse, so dass regelmäßig bei unterschiedlichen Anlagen auch unterschiedliche Substratzusammensetzungen eingesetzt werden.

Ein gattungsgemäßes Verfahren zum kontinuierlichen, einstufigen Betrieb einer Biogasanlage zur Erzeugung von Biogas durch anaerobe Nassvergärung ist beispielsweise aus der Schrift "Handreichung, Biogasgewinnung und -nutzung, Bundesministerium für Verbraucherschutz, Ernährung und Landwirtschaft, Fachagentur nachwachsende Rohstoffe e. V." bekannt. Hier wird auch auf die üblichen Probleme in der Anfahrphase bei neu gebauten Biogasanlagen oder bei einem Neuanlauf von Biogasanlagen hingewiesen. Diese Probleme liegen in einer anfänglich sehr geringen Gasausbeute und/oder zu hohen Säurekonzentrationen, was im schlimmsten Fall den kompletten Austausch des Fermenterinhalts und ein erneutes Anfahren des Fermentationsprozesses mit erheblichen wirtschaftlichen Verlusten nach sich ziehen kann.

Es wird zwar vorgeschlagen, beim Anfahren des Fermentationsprozesses ein Animpfen mit vergorener Rindergülle vorzunehmen, da darin bereits die für den Fermentationsprozess benötigten Bakterien vorhanden sind. Deren Anzahl und deren Aktivität sind aber vorerst nur sehr gering, so dass in der Anfahrphase einerseits nur wenig Substrat mit einer geringen Raumbelastung zugegeben werden darf, so dass andererseits nur wenig Biogas erzeugt wird. Diese Zugabe soll nur langsam und in kleinen Schritten erhöht werden, um eine substratspezifische Entwicklung von Bakterien zu fördern und insbesondere den Methanbakterien genügend Zeit für ihr Wachstum zu geben. Bis zu einem stabilen Prozess des Substratabbaus vergeht hier relativ viel Zeit mit geringer Biogasausbeute und damit ungünstig geringer Energieerzeugungsrate. Zudem besteht die Gefahr, dass im relativ instabilen Anfahrprozess die vorstehenden Probleme mit hohen Säurekonzentrationen und geringer Gasausbeute mit einer Versäuerung des gesamten Reaktorinhalts auftreten, so dass jedenfalls während der Anfahrphase ein hoher Aufwand für eine intensive Prozessbeobachtung und -führung erforderlich ist.

Weiter ist es auch bekannt, dass die vorstehenden Probleme entsprechend bei einer Veränderung in der Zusammensetzung des zugeführten Substrats auftreten, was einer Milieuumstellung für die Bakterien und damit etwa der vorstehenden Anfahrphase bei einer Neubeschickung entspricht. Plötzliche Veränderungen in der Substratzusammensetzung wirken sich besonders stark durch eine reduzierte Gasproduktion aus. Es wird daher auch hierzu vorgeschlagen, solche Änderungen in der Substratzusammensetzung nur allmählich und zeitlich gestreckt durchzuführen, um einen eingefahrenen stabilen Prozess nicht zu gefährden. Eine solche empfohlene allmähliche Umstellung ist in der Praxis oft nicht oder nur mit erheblichem Aufwand durchzuführen, insbesondere dann, wenn in der vorhandenen Biogasanlage saisonal bedingte oder durch Zulieferverträge bedingte Substratumstellungen schnell erforderlich sind, so dass eine reduzierte Gasausbeute und eine Gefährdung des stabilen Prozessablaufs in Kauf genommen werden müssen.

Aufgabe der Erfindung ist es, ein gattungsgemäßes Biogasanlagen-Betriebsverfahren zum einstufigen Betrieb einer Biogasanlage so weiterzubilden, dass in einer Anfahrphase mit einem Substrat einer vorgegebenen Substratzusammensetzung die Biogaserzeugungsrate erhöht wird und schnell eine stabile Prozessführung erreicht wird. Unter dem Begriff Anfahrphase wird hier sowohl ein Neustart nach einer Neubeschickung mit einem Substrat einer vorgegebenen Substratzusammensetzung verstanden, als auch im eingefahrenen Betrieb der weitere Betrieb nach einem Wechsel von einer anderen Substratzusammensetzung zu der vorgegebenen Substratzusammensetzung.

Diese Aufgabe wird mit den Merkmalen des Anspruchs 1 gelöst.

Gemäß Anspruch 1 erfolgt in der Anfahrphase eine Zudosierung von substratspezifischen Bakterien, die unabhängig von der Biogasanlage in einem Züchtungsreaktor aus einem Substrat der vorgegebenen Substratzusammensetzung vorgezüchtet worden sind.

Die Erfindung macht sich zwei Gegebenheiten zunutze: Einerseits entwickeln sich geeignete Bakterien für die Nassvergärung substratspezifisch. D. h., dass sich bei einer Nassvergärung mit einem vorgegebenen Substrat bei genügend langen Entwicklungszeiten und guten Randbedingungen, insbesondere bei geeigneten Temperaturen und guter Durchmischung im Züchtungsreaktor, vorrangig die Bakterien entwickeln, die für einen optimalen Abbau des gerade vorliegenden Substrats mit dessen vorgegebener Substratzusammensetzung besonders gut geeignet sind. Diese Bakterien führen damit auch zu einer besonders hohen Biogaserzeugungsrate.

Andererseits ist dem Betreiber einer Biogasanlage regelmäßig die Substratzusammensetzung für seine Anlage vor einem Neustart oder vor einem Wechsel der Substratzusammensetzung bekannt, so dass ein solches Substrat vorher bereits zur Züchtung substratspezifischer Bakterien verfügbar ist.

Wenn gemäß Anspruch 1 bereits eine vorgezüchtete Menge substratspezifischer Bakterien in einer Anfahrphase in den Biogasfermenter zugegeben werden, wird schon von Anfang an eine relativ hohe Biogaserzeugungsrate erreicht. Der Aufbau der geeigneten Bakterienpopulationen fängt dann dadurch nicht von ganz unten an, sondern entsprechend einem fliegenden Start schon auf relativ hohem Niveau, wodurch auch vorteilhaft die langsame, ähnlich wie bei Kettenbriefen oder dem Schneeballeffekt ablaufende, Initialaufbauphase nicht in der Biogasanlage durchgeführt werden muss.

Der Anfahrvorgang bei einem Neustart oder einer Änderung der Substratzusammensetzung ist daher vergleichsweise stabil mit reduzierten Problemen hinsichtlich hoher Säurekonzentrationen. Die Anfahrphase kann zeitlich wesentlich kürzer gehalten werden und ein stabiler wirtschaftlich lukrativer Betrieb der Biogasanlage mit der vorgegebenen Substratzusammensetzung ist somit früher möglich. Die bekannten Probleme, dass eine neu angefahrene Biogasanlage möglicherweise erst nach Monaten eine optimal hohe Gasausbeute hat, sind hier praktisch ausgeschlossen.

Die bekannten Probleme und Risiken in Verbindung mit einem Neustart oder einer Änderung der Substratzusammensetzung sind beim vorliegenden Verfahren im wesentlichen von der kritischen (großen) Biogasanlage zeitlich vorverlegt in den unkritischen Züchtungsreaktor, welcher wesentlich kleiner im Vergleich zum Biogasfermenter ausgeführt sein kann und der unabhängig vom Biogasfermenter betrieben wird. Bei tatsächlich auftretenden Problemen bezüglich eines vorgegebenen Substrats sind diese in einem Züchtungsreaktor relativ einfach beherrschbar und wirken sich vor allem wirtschaftlich nur unbedeutend aus.

Gemäß Anspruch 2 werden die benötigten substratspezifischen Bakterien dergestalt gezüchtet, dass
- ein Züchtungssubstrat hergestellt/beschafft wird, das in der Zusammensetzung dem Substrat entspricht, mit dem die Biogasanlage in einer zeitlich nachgeordneten Anfahrphase betrieben werden soll.
- Dieses Substrat wird als Züchtungssubstrat in den Züchtungsreaktor eingebracht. Gegebenenfalls kann zum Start der Vergärung das Züchtungssubstrat mit einem Impfsubstrat, beispielsweise aus vergorener Rindergülle, angeimpft werden.
- Die Reaktortemperatur im Reaktorinnenbereich wird auf einen mesophilen oder thermophilen Temperaturbereich, vorzugsweise über eine geregelte Heizung, eingestellt, wodurch nach dem Verschließen des Züchtungsreaktors der Prozess einer anaeroben Nassvergärung anläuft.
- Schrittweise und langsam wird die Menge des Züchtungssubstrats im Züchtungsreaktor erhöht, wobei innerhalb einer Entwicklungszeit geeignete Bakterien, insbesondere substratspezifische Methanbakterien, vorzugsweise bis zu einem stabilen eingefahrenen Vergärungsabbau des Züchtungssubstrats erzeugt werden. Nun steht ein Dosiersubstrat mit einer relativ großen Menge darin enthaltener substratspezifischer Bakterien zur Verfügung.
- Das Dosiersubstrat kann nun ganz oder teilweise aus dem Züchtungsreaktor entnommen werden und einem Biogasfermenter während einer Anfahrphase, vorzugsweise chargenweise zugeführt werden.

Das angegebene Züchtungsverfahren ist relativ einfach durchführbar, wobei es darauf ankommt, im Züchtungsreaktor möglichst gleiche Milieubedingungen für die Bakterien herzustellen, wie sie später im Biogasfermenter der Biogasanlage vorliegen, um optimal geeignete, substratspezifische Bakterienpopulationen zu erzeugen.

Mit Anspruch 3 wird eine Kolonne aus mehreren Züchtungsreaktoren vorgeschlagen, so dass parallel unterschiedliche Dosiersubstrate mit jeweils zugeordneten substratspezifischen Bakterien für den Einsatzbedarf erzeugt und vorgehalten werden können. In einem Züchtungsreaktor kann beispielsweise ein Dosiersubstrat für einen Substratmix aus vorwiegend Schweinegülle und Hühnermist einer bestimmten prozentualen Zusammensetzung hergestellt werden, während im nächsten Züchtungsreaktor ein Dosiersubstrat für einen Substratmix aus Maissilage und Rindergülle bestimmter prozentualer Zusammensetzung mit den jeweils optimal geeigneten substratspezifischen Bakterien erzeugt wird. Dadurch können am Aufstellungs- und Betriebsort der Kolonne Dosiersubstrate für weit auseinander liegende und gegebenenfalls auch zeitlich versetzt anzufahrende Biogasanlagen hergestellt werden. Zweckmäßig ist nach Anspruch 4 ein so hergestelltes Dosiersubstrat flüssig und pumpfähig und wird in wärmegedämmten und auf Temperatur gehaltenen Behältern aufgenommen und transportiert bis zu einer Zudosierung in einen Biogasfermenter.

Alternativ zur vorstehenden Kolonnenanordnung mehrerer Züchtungsreaktoren kann es nach Anspruch 5 gegebenenfalls vorteilhaft sein, einen Züchtungsreaktor nahe an einem Biogasfermenter praktisch als Bestandteil der Biogasanlage anzuordnen. Der Züchtungsreaktor kann zur Einbringung von Dosiersubstrat mit dem Biogasfermenter über eine absperrbare Pumpleitung verbunden sein. Eine solche Anordnung ist zweckmäßig, wenn bedingt durch saisonal unterschiedliche nachwachsende Rohstoffe und/oder vertragliche Abnahmebedingungen für in Zeitabständen wechselnde zugelieferte Biomassen, die Biogasanlage öfter an wechselnde Substratzusammensetzungen angepasst oder gegebenenfalls neu gestartet werden muss. Für solche Fälle kann dann jeweils schon im Vorgriff auf die Umstellung im Züchtungsreaktor der Züchtungsprozess für substratspezifische Bakterien vorgenommen werden. Auch hier können somit im Vorfeld im Züchtungsreaktor die späteren Milieubedingungen der großen kritischen Biogasanlage vorweg eingestellt werden und geeignete Bakterien gezüchtet werden. Da hier der Züchtungsreaktor örtlich in der Nähe des Biogasfermenters angeordnet ist, kann nach Anspruch 6 für einen kostengünstigen Aufbau die ohnehin vorhandene Regeleinrichtung der Biogasanlage weitgehend auch für den Züchtungsreaktor mitverwendet werden.

Nach Anspruch 7 wird das Dosiersubstrat in einem Vorratsbehälter am Biogasfermenter vorgehalten und chargenweise zudosiert, wobei mittels einer Rühreinrichtung das Dosiersubstrat in das Anlagensubstrat eingerührt wird. Dazu können gegebenenfalls ohnehin vorhandene Vorratsbehälter und Anlagenteile mitverwendet werden. Je nach den Gegebenheiten kann aber der Dosiervorgang auch anders durchgeführt werden, beispielsweise durch gezielte Zugabe zu Trockensubstanzen oder in Verbindung mit dem Einspülen von Verdünnungsflüssigkeiten.

Nach Anspruch 8 soll der Züchtungsreaktor im Verhältnis zum Biogasfermenter klein ausgeführt sein. Die Größenverhältnisse sollen jedoch dabei so gewählt werden, dass im Züchtungsreaktor noch eine Abbildung der späteren Milieuverhältnisse des Biogasfermenters in Verbindung mit einer merklichen Menge von gezüchteten Bakterien möglich ist.

Aus den Merkmalen des Anspruchs 9 ist ersichtlich, dass es eine Vielzahl unterschiedlicher Substrate gibt, die nach Art der Biomassen und deren prozentualer Zusammensetzung sehr unterschiedlich sein können, was deutlich zeigt, dass nicht alle Bakterien gleichermaßen für alle unterschiedlichen Substrate geeignet sein können. Somit ergibt die erfindungsgemäße frühzeitige Zugabe substratspezifischer Bakterien erhebliche Vorteile hinsichtlich der Gasausbeute und eines stabilen Prozessverlaufes.

Anhand einer Zeichnung wird die Erfindung näher erläutert.

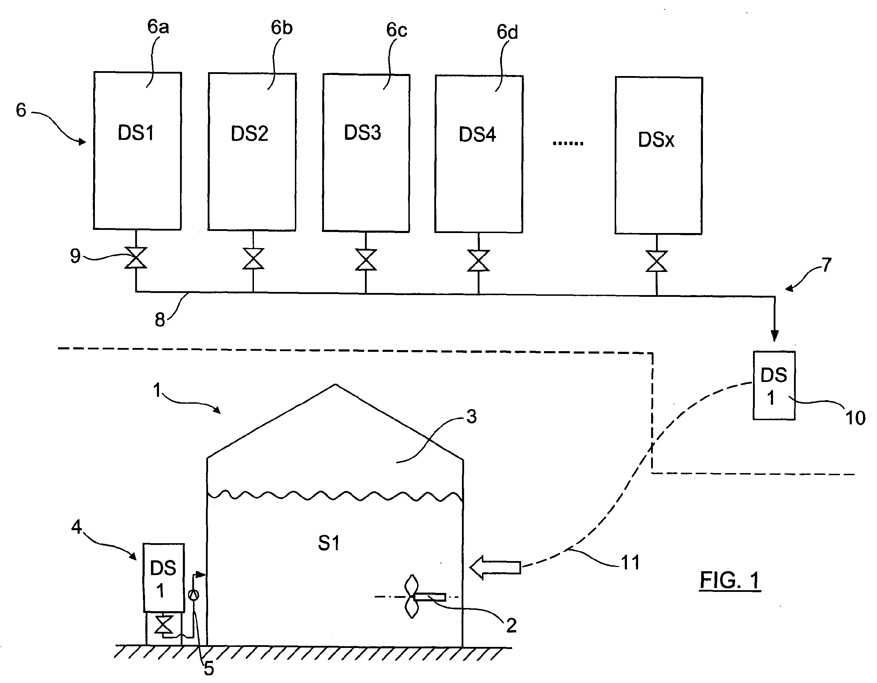

In Fig. 1 sind zwei alternative Anordnungen von Züchtungsreaktoren und eine Biogasanlage schematisch dargestellt.

In Fig. 1 ist ein Biogasfermenter 1 als Teil einer Biogasanlage schematisch dargestellt, der mit einem Substrat S1 gefüllt und betrieben wird. Zudem ist schematisch eine Rühreinrichtung 2 gezeigt. Oberhalb des Substratspiegels ist der Biogasfermenter durch ein Dach luftdicht abgedeckt, unter dem sich Biogas 3 sammelt, welches durch (nicht dargestellte) Gasleitungen einem Blockheizkraftwerk zugeführt wird. Die Anlage soll sich gerade in der Einfahrphase befinden, wobei entsprechend dem vorstehenden Verfahren Dosiersubstrat DS1 chargenweise zudosiert wird.

Die Herstellung des Dosiersubstrats DS1 kann in einer ersten Alternative in einem Züchtungsreaktor 4 erfolgen, der hier in unmittelbarer Nähe des Biogasfermenters 1 angeordnet ist und der über eine absperrbare Pumpleitung 5 mit dem Biogasfermenter 1 verbunden ist.

Alternativ oder gegebenenfalls zusätzlich kann an einem anderen entfernten Ort eine Kolonne 6 aus mehreren Züchtungsreaktoren 6a, 6b, 6c, 6d aufgestellt sein, wovon ein Züchtungsreaktor 6a für die Herstellung des benötigten Dosiersubstrats verwendet wird. Zur besseren Übersichtlichkeit sind die Züchtungsreaktoren 6a, 6b, 6c, 6d und deren Abfülleinrichtung 7 im Vergleich zum Biogasfermenter 1 vergrößert dargestellt. In den anderen Züchtungsreaktoren 6b, 6c, 6d können gleichzeitig oder zeitversetzt andere Dosiersubstrate DS2, DS3, DS4 für andere Substratzusammensetzungen für andere Biogasanlagen hergestellt werden. Zur Herstellung einer gemeinsamen Abfülleinrichtung 7 sind die Züchtungsreaktoren 6a, 6b, 6c, 6d an eine Sammelleitung 8 über steuerbare Ventile 9 angeschlossen. An der Abfülleinrichtung 7 wird hier das Dosiersubstrat DS1 in einen wärmegedämmten Behälter 10 abgefüllt und für eine Zudosierung zum Biogasfermenter 1 transportiert (Wegpfeil 11).

## Patentansprüche

1. Biogasanlagen-Betriebsverfahren zum einstufigen Betrieb einer Biogasanlage zur Erzeugung von Biogas durch anaerobe Nassvergärung aus einem dem Biogasfermenter der Biogasanlage zugeführten Substrat einer vorgegebenen Substratzusammensetzung,
**dadurch gekennzeichnet,**
**dass** in einer Anfahrphase mit dem Substrat (S1) der vorgegebenen Substratzusammensetzung, als Neustart nach einer Neubeschickung oder im eingefahrenen Betrieb nach einer Änderung der Substratzusammensetzung, eine Zudosierung von substratspezifischen Bakterien erfolgt, die unabhängig von der Biogasanlage in einem Züchtungsreaktor (4; 6a) mittels eines Substrats (S1) der vorgegebenen Substratzusammensetzung vorgezüchtet worden sind.

2. Biogasanlagen-Betriebsverfahren nach Anspruch 1, **dadurch gekennzeichnet, dass** die Züchtung der substratspezifischen Bakterien mit folgenden Verfahrensschritten durchgeführt wird:
- Herstellung/Beschaffung eines Züchtungssubstrats, das in der Zusammensetzung dem Substrat (S1) entspricht, mit dem die Biogasanlage in einer zeitlich nachgeordneten Anfahrphase betrieben werden soll,
- Einbringen eines Teils des Züchtungssubstrats in den Züchtungsreaktor (4; 6a),
- Einstellung der Reaktortemperatur im Reaktorinnenbereich auf einen mesophilen oder thermophilen Temperaturbereich entsprechend der nachgeordneten Anfahrphase in der Biogasanlage, und Start einer anaeroben Nassvergärung,
- schrittweise, langsame Erhöhung der Menge des Züchtungssubstrats im Züchtungsreaktor und somit Erhöhung der Raumbelastung innerhalb einer Entwicklungszeit für eine Entwicklung und Züchtung substratspezifischer Bakterien, insbesondere für ein Wachstum substratspezifischer Methanbakterien vorzugsweise bis zu einem stabilen eingefahrenen Vergärungsabbau des Züchtungssubstrats, wonach ein Dosiersubstrat (DS1) mit darin in großer Menge enthaltenen substratspezifischen Bakterien vorliegt,
- Entnahme zumindest eines Teils des Dosiersubstrats (DS1) mit den darin enthaltenen substratspezifischen Bakterien aus dem Züchtungsreaktor (4; 6a),
- Transport des Dosiersubstrats (DS1) zum Biogasfermenter (1) der Biogasanlage und Zudosierung in den Biogasfermenter (1) während der dortigen mit dem vorgegebenen Substrat laufenden Anfahrphase.

3. Biogasanlagen-Betriebsverfahren nach Anspruch 2, **dadurch gekennzeichnet, dass** in einer Kolonne (6) von Züchtungsreaktoren (6a, 6b, 6c, 6d) jeweils eine Beschickung mit unterschiedlichen vorgegebenen Substraten erfolgt, so dass jeweils zugeordnete unterschiedliche Dosiersubstrate (DS1, DS2, DS3, DS4) mit substratspezifischen Bakterien erzeugt und für den Einsatzbedarf vorgehalten werden.

4. Biogasanlagen-Betriebsverfahren nach einem der Ansprüche 1 bis 3, **dadurch gekennzeichnet, dass** die substratspezifischen Bakterien in einem flüssigen, pumpfähigen Dosiersubstrat (DS1) enthalten sind, das bis zur Zudosierung in einem wärmegedämmten, im mesophilen oder thermophilen Temperaturbereich gehaltenen Behälter (10) aufgenommen ist.

5. Biogasanlagen-Betriebsverfahren nach einem der Ansprüche 1 bis 4, **dadurch gekennzeichnet, dass** der Züchtungsreaktor (4) in der Nähe des Biogasfermenters (1) angeordnet ist und gegebenenfalls über eine absperrbare Pumpleitung (5) damit verbunden ist.

6. Biogasanlagen-Betriebsverfahren nach Anspruch 5, **dadurch gekennzeichnet, dass** die Regeleinrichtung der Biogasanlage für deren Züchtungsreaktor (4) mitverwendet wird.

7. Biogasanlagen-Betriebsverfahren nach einem der Ansprüche 1 bis 6, **dadurch gekennzeichnet, dass** Dosiersubstrat (DS1) in einem Vorratsbehälter am Biogasfermenter (1) vorgehalten und chargenweise zudosiert, sowie mittels einer Rühreinrichtung (2) eingerührt wird.

8. Biogasanlagen-Betriebsverfahren nach einem der Ansprüche 1 bis 7, **dadurch gekennzeichnet, dass** der Züchtungsreaktor (4; 6a) klein im Verhältnis zum Biogasfermenter (1) ist.

9. Biogasanlagen-Betriebsverfahren nach einem der Ansprüche 1 bis 8, **dadurch gekennzeichnet, dass** das Substrat mit der vorgegebenen Substratzusammensetzung ein Substratmix aus einer Mischung von Wirtschaftsdüngern, als Gülle und/oder Mist, und/oder von nachwachsenden Rohstoffen und/oder von organischen Substraten der weiterverarbeitenden Agrarindustrie und/oder von organischen Reststoffen aus Kommunen und/oder von Schlachtrückständen ist.
